# DEMANDE DE BREVET EUROPEEN

(11) **EP 0 773 274 A1**
(43) Date de publication de la demande: **14.05.1997**
(21) Numéro de dépôt: 96402265.1
(22) Date de dépôt: 24.10.1996
(51) Int. Cl.: C10G 2/00, C07C 1/06

(54) **Procédé de conversion de gaz de synthèse en hydrocarbures**

(30) Priorité: 10.11.1995 FR 9513338
(71) Demandeur: INSTITUT FRANCAIS DU PETROLE, 92502 Rueil-Malmaison (FR)
(72) Inventeur: Chaumette, 78380 Bougival (FR); Boucot, Pierre, 69360 Ternay (FR); Morel, 69340 Francheville (FR)

(57) **Abrégé**

L'invention concerne un procédé de synthèse d'hydrocarbures essentiellement linéaires et saturés, contenant au moins 80 % poids d'hydrocarbures C₅⁺ par rapport à l'ensemble des hydrocarbures formés, à partir d'un gaz de synthèse CO-(CO₂)-H₂, la conversion du gaz de synthèse en hydrocarbures étant opérée sous une pression totale comprise entre 0,1 et 15 MPa, la température étant comprise entre 150 et 350° C, la vitesse volumique horaire étant comprise entre 100 et 30 000 volumes de gaz de synthèse par volume de catalyseur et par heure, et le rapport molaire H₂/CO dans le gaz de synthèse étant compris entre 1:2 et 5:1, ledit procédé étant caractérisé en ce que l'on opère dans une zone réactionnelle en lit catalytique bouillonnant en présence d'un catalyseur comprenant au moins un métal du groupe VIII, et en présence d'une phase liquide.

## Description

La présente invention concerne un procédé de synthèse d'hydrocarbures à partir d'un mélange CO-(CO₂)-H₂, c'est-à-dire d'un mélange CO-H2 comprenant éventuellement du CO₂ appelé gaz de synthèse. Elle concerne plus particulièrement l'utilisation d'une technologie (le lit catalytique bouillonnant aussi nommé lit fluidisé triphasique) en présence d'une phase liquide et d'une formulation catalytique permettant de réaliser la conversion du gaz de synthèse en un mélange d'hydrocarbures linéaires et saturés essentiellement constitué d'hydrocarbures C₅⁺ (c'est-à-dire possédant au moins 5 atomes de carbone par molécule), ou plus précisement en un mélange d'hydrocarbures essentiellement linéaires et saturés, contenant au moins 80 % poids d'hydrocarbures C₅⁺ par rapport à l'ensemble des hydrocarbures formés, utilisables en tant que carburant ou combustible liquide.

Il est connu de l'homme du métier que le gaz de synthèse peut être converti en hydrocarbures en présence de catalyseurs contenant des métaux de transition. Cette conversion, opérée en température et sous pression, est connue dans la littérature sous le nom de synthèse FISCHER- TROPSCH. Ainsi des métaux du groupe VIII de la classification périodique des éléments tel que le fer, le ruthénium, le cobalt et le nickel catalysent la transformation de mélanges CO-(CO₂)-H₂ en hydrocarbures liquides et/ou gazeux.

Les produits préparés par synthèse FISCHER- TROPSCH en présence de catalyseurs comprenant des métaux du groupe VIII présentent une distribution très large en terme de poids moléculaire. Ainsi seulement une faible proportion des produits obtenus se situent dans la gamme des distillats moyens constitués par des fractions kérosène et gasoil, la (ou les) fraction(s) kérosène étant constituée(s) par un mélange d'hydrocarbures dont les points d'ébullition sont compris entre 140° et 300°C, et la (ou les) fraction(s) gasoil étant constituée(s) par un mélange d'hydrocarbures de points d'ébullition compris entre 180° et 370°C lors d'une distillation atmosphérique telle que réalisée par l'homme du métier sur un brut pétrolier.

Des efforts importants ont été entrepris depuis 1973 afin d'améliorer le rendement en distillats moyens des procédés basés sur la conversion du gaz de synthèse. En particulier, des catalyseurs à base de cobalt ont été utilisés. Ainsi, le catalyseur décrit dans le brevet US-A-5.302.622, comprenant du cobalt, du cuivre et du ruthénium et préparé par gélification, conduit à l'obtention d'un mélange d'hydrocarbures essentiellement linéaires et saturés contenant au moins 80% poids d'hydrocarbures C₅⁺ par rapport à l'ensemble des hydrocarbures formés. De même, le catalyseur décrit dans le brevet français FR-2.677.992, contient du cobalt, au moins un élément M additionnel (par exemple sous forme métallique ou sous forme d'oxyde) choisi dans le groupe constitué par le molybdène et le tungstène et au moins un élément N additionnel (par exemple sous forme métallique ou sous forme d'oxyde) choisi dans le groupe constitué par les éléments des groupes la, lla, lb (tels que par exemple le sodium, le potassium, le magnésium, le calcium, le cuivre ou l'argent), le ruthénium, le palladium, l'uranium, le praséodyme et le néodyme, de préférence dans le groupe formé par le sodium, le potassium, le ruthénium, le cuivre et l'uranium, l'ensemble de ces éléments étant dispersés sur un support, qui est de préférence constitué par au moins un oxyde d'au moins un élément choisi dans le groupe formé par les éléments suivants: Si, Al, Ti, Zr, Sn, Zn, Mg, Ln (où Ln est une terre rare). Enfin, la demande de brevet français 95/11.296 du 25/09/95 concerne un catalyseur comprenant un support comportant au moins un oxyde d'élément Si, Al, Ti, Zr, Sn, Zn, Mg ou Ln (où Ln est une terre rare), du cobalt, au moins un élément A choisi dans le groupe formé par le ruthénium, le platine, le palladium et l'uranium, au moins un élément B choisi dans le groupe formé par le molybdène et le tungstène. Sa préparation particulière comprend au moins les étapes successives suivantes :
- -(1): la formation d'un précurseur comprenant au moins le cobalt et au moins une partie du support
- -(2): la réduction au moins partielle dudit précurseur en présence d'au moins un composé réducteur, et
- -(3): le dépôt sur le précurseur réduit de toute partie de composé présent dans le catalyseur et non présent dans le précurseur.

D'autre part, les procédés de synthèse Fischer-Tropsch sont des procédés opérant généralement en phase gazeuse ou en plase liquide. Le lit catalytique disposé dans le réacteur de synthèse Fischer-Tropsch est en général soit fixe, soit circulant, soit fluidisé ou expansé.

Il avait été envisagé il y a 15 ans d'utiliser pour la mise en oeuvre de la synthèse Fischer-Tropsch d'opérer dans un réacteur dans lequel le lit catalytique était opéré en lit bouillonnant. Ainsi NL 7.708.307 et FR 2.490.668 mentionnent de façon très générale, l'utilisation possible de la mise en oeuvre en lit bouillonnant. D'autre part, il a été envisagé de façon plus précise de mettre en oeuvre un réacteur dans lequel le lit catalytique est opéré en lit bouillonnant, en présence d'un catalyseur à base de fer, traité au soufre (US-A-4.242.234 et US-A-4.256.654) ou au chlore (US-A-4.172.842 et US-A-4.252.685) et opéré à haute température (au moins 300°C), de façon à maximiser le rendement en hydrocarbures légers. Dans ces mises en oeuvre, le lit catalytique est expansé et fluidisé au moyen d'un débit de gaz suffisamment élevé. Aucune phase liquide n'est introduite ou recyclée dans la section réactionnelle en dehors des produits de la réaction. Les calories produites par la réaction sont évacuées au moyen d'échanges internes et de la phase gazeuse.

La présente invention concerne un procédé de synthèse d'hydrocarbures essentiellement linéaires et saturés, contenant au moins 80 % poids d'hydrocarbures C₅⁺ par rapport à l'ensemble des hydrocarbures formés, à partir d'une charge comprenant du monoxyde de carbone CO, de l'hydrogène H₂ et éventuellement du dioxyde de carbone CO₂, appelée gaz de synthèse, ledit procédé étant caractérisé en ce que l'on opère dans une zone réactionnelle en lit catalytique bouillonnant en présence d'un catalyseur comprenant au moins un métal du groupe VIII de la classification périodique des éléments, de préférence choisi dans le groupe formé par le fer et le cobalt, de façon encore plus préférée le cobalt, et en présence d'une phase liquide.

Ce procédé présente l'avantage d'éliminer la chaleur produite par la réaction de façon plus efficace et donc d'éviter une trop forte augmentation de la température au niveau du grain de catalyseur. Il en découle des performances améliorées en synthèse d'hydrocarbures C₅⁺.

Les conditions de mise en oeuvre du procédé selon l'invention sont habituellement les suivantes.

Le mélange est chargé dans la zone réactionnelle dans un rapport molaire H₂ / CO compris entre 1:2 et 5:1, de préférence entre 1,2:1 et 2,5:1. Ledit mélange peut en outre comprendre du bioxyde de carbone CO₂ et éventuellement d'autres impuretés tels que des hydrocarbures (méthane, éthane, propane et butanes). La température de réaction est comprise entre 150 et 350°C, de préférence entre 170 et 300°C et de manière encore plus préférée entre 180 et 280°. La pression est comprise entre 0,1 et 15 MPa, de préférence entre 1 et 10 MPa. Enfin, la vitesse volumétrique horaire est comprise entre 100 et 30.000, de préférence entre 400 et 10.000 volumes de gaz de synthèse par volume de catalyseur et par heure. La vitesse du liquide, c'est-à-dire de la phase liquide en présence de laquelle la synthèse est réalisée, est comprise entre 0,1 et 10 volume de liquide par volume de catalyseur et par heure de préférence entre 0,2 et 5 volume de liquide par volume de catalyseur et par heure.

La phase liquide en présence de laquelle le procédé se déroule est initialement introduite dans la zone réactionnelle afin de jouer le rôle de fluide caloporteur et d'expanser le lit catalytique. Puis, une fois que la réaction a démarré, il est préféré dans le cadre de la présente invention de recycler au moins une partie de l'effluent de la zone réactionnelle dans ladite zone, éventuellement après fractionnement, de façon à obtenir ladite phase liquide. Le rapport entre le volume de liquide recyclé et le volume de charge est généralement compris environ entre 0,5:1 et au moins 50:1, de façon préférée entre 2:1 et 10:1.

Dans le cadre de la présente invention, il est aussi possible d'utiliser au moins un moyen de transfert de chaleur, généralement sous forme d'échangeur, de façon à éliminer au moins une partie de la chaleur produite par la réaction.

Le catalyseur utilisé dans le procédé selon l'invention est un catalyseur comprenant au moins un métal du groupe VIII, de préférence choisi dans le groupe formé par le fer et le cobalt, de façon encore plus préférée le cobalt, bien connu par l'homme du métier pour réaliser la synthèse Fischer-Tropsch. Par exemple les catalyseurs dont la formulation est décrite dans les brevets US-A-5.302.622 et FR-2.677.992 et la demande de brevet français 95/11.296 peuvent être utilisés de façon encore plus préférée. La taille du catalyseur est adaptée à son utilisation dans un lit catalytique bouillonnant. Quand le diamètre équivalent moyen des particules catalytiques constituant le catalyseur est compris entre 100 et 5000 µm (1 µm= 1. 10⁻⁶ m = 1 micron ), de préférence entre 350 et 3000 µm, la vitesse superficielle de liquide ascendant est généralement comprise entre 0,5.10⁻³ et 15 m/s, de préférence entre 0,1 et 10 m/s. Dans une telle situation, le catalyseur est périodiquement soutiré usé de la zone réactionnelle tandis que du catalyseur frais est ajouté dans ladite zone. Mais il est aussi envisageable d'utiliser une opération, de préférence à une seule passe, avec un catalyseur ajouté avec la charge. Dans la zone réactionnelle, qui est généralement un réacteur, la densité des particules catalytiques, la vitesse du liquide ascendant et l'effet ascendant des gaz (hydrogène et monoxyde de carbone principalement) sont des facteurs importants pour l'expansion et l'utilisation du lit catalytique. Par contrôle de la taille et de la densité des particules catalytiques et des vitesses des gaz et liquides, en tenant compte de la viscosité du liquide et des conditions opératoires, le lit catalytique s'expanse jusqu'à une hauteur contrôlée.

La réaction de synthèse Fischer-Tropsch, permettant la conversion du gaz de synthèse (mélange d'hydrogène et d'oxyde(s) de carbone), est très exothermique. C'est pourquoi de grandes quantités de chaleur doivent être extraites de la zone réactionnelle. La zone réactionnelle, généralement au moins un réacteur, les réacteurs étant en série dans le cas où la zone réactionnelle comprend au moins deux réacteurs, comprend une arrivée de charge chaude et une grille qui supporte le catalyseur, de façon à ce que le liquide et le gaz qui sont chargés dans le réacteur, traversant le réacteur dans le sens ascendant, expansent le lit catalytique à partir de 10 et jusqu'à 50 % par rapport à la hauteur dudit lit au repos, et placent le catalyseur en mouvement aléatoire dans le liquide. La charge et le liquide de recyclage sont chargés dans la zone réactionnelle à une vitesse suffisante pour fournir une vitesse superficielle comprise entre 0,5. 10⁻³ et 5 m/s, de préférence entre 0,1 et 10 m/s, de telle façon que le lit est expansé et maintenu dans des conditions bouillonnantes par le flux liquide qui remonte à travers le réacteur. Une telle ébullition du lit catalytique produit un excellent transfert de chaleur à travers le lit catalytique, prévenant la formation de points chauds et aidant au transfert de chaleur depuis chaque point du lit jusqu'au(x) moyen(s) de transfert de chaleur éventuellement présent(s) dans la zone réactionnelle. Pour les lits bouillonnants pour lesquels de grands transferts de chaleur doivent être effectués, de tels moyens sont typiquement des tubes de génération de vapeur ou des spires, ou tout autre moyen connu de l'homme du métier. La réaction de synthèse Fischer-Tropsch est menée dans une phase liquide, généralement hydrocarbonée, de préférence comprenant au moins 5, de manière encore plus préférée au moins 10, atomes de carbone par molécule. D'une manière plus préférée on utilise une fraction de l'effluent issu de la zone réactionnelle.

Le catalyseur, chargé dans la zone réactionnelle est soumis à une préréduction avant utilisation, par au moins un composé réducteur, par exemple choisi dans le groupe formé par l'hydrogène, le monoxyde de carbone et l'acide formique, éventuellement mis en contact avec un gaz inerte (azote par exemple) en rapport molaire composé réducteur/(composé réducteur + gaz inerte) compris entre 0,001:1 et 1:1.

La préréduction est menée entre 150 et 600°C, de préférence entre 200 et 500°C, entre 0,1 et 10 MPa et à une vitesse volumétrique horaire comprise entre 100 et 40 000 volumes de mélange par volume de catalyseur et par heure. Cette préréduction sera préférentiellement menée en phase liquide comprenant au moins un hydrocarbure ayant au moins 5, de préférence au moins 10, atomes de carbone par molécule.

La conversion du gaz de synthèse en hydrocarbures est ensuite opérée selon les conditions opératoires indiquées précédemment.

Les catalyseurs comprenant au moins un métal du groupe VIII, de préférence choisi dans le groupe formé par le fer et le cobalt, de façon encore plus préférée le cobalt, utilisés selon l'invention, sont particulièrement actifs et stables dans la réaction de synthèse d'hydrocarbures à partir de gaz de synthèse. Lesdits catalyseurs permettent d'obtenir des hydrocarbures essentiellement paraffiniques, dont la fraction présentant les points d'ébulition les plus élevés peut être convertie avec un rendement élevé en distillats moyens (coupes gasoil et kérosène) par un procédé d'hydroconversion tel que l'hydrocraquage et/ou l'hydroisomérisation catalytiques.

La figure illustre un mode de réalisation du procédé selon l'invention.

Sur la figure, le gaz de synthèse **1** comprimé (mélange CO,CO₂,H₂) est préchauffé, par passage dans un four de préchauffage **2**, à une température variable selon la température à laquelle est effectuée la réaction. ll est également possible de préchauffer le gaz de synthèse au moyen d'un échangeur charge effluent. Le gaz de synthèse préchauffé **3** est introduit dans le réacteur à lit bouillonnant **5**. Une fraction hydrocarbonée liquide **17** (coupe gasoil) est également introduite dans le réacteur **5** par le moyen d'une pompe **16** avec préchauffage dans le four **15**.

Le réacteur **5** possède un distributeur interne **4** qui rempli également le rôle de support de catalyseur. Ainsi le liquide introduit dans le réacteur permet d'expanser le lit catalytique **12** d'environ au moins 10% et habituellement d'environ 50% (niveau de catalyseur **10**) au dessus de sa hauteur au repos et de disperser le catalyseur de manière aléatoire dans la phase liquide. Ce réacteur est décrit dans le brevet U.S-A-Re 25,770.

Selon la granulométrie du catalyseur, ce dernier est introduit dans le réacteur soit en suspension dans la fraction liquide (slurry), soit par l'alimentation spécifique **6**. Une partie du catalyseur peut également être déchargée par le soutirage **13**.

Le catalyseur utilisé est tel que décrit précédemment.

Il est généralement nécessaire de recycler du liquide situé au dessus du niveau du catalyseur **10** vers le plateau distributeur **4**, afin de générer un flux de liquide suffisant pour maintenir le catalyseur en mouvement dans la phase liquide et de favoriser la réaction. La recirculation du liquide est préférentiellement accomplie au moyen de la conduite centrale **11**, qui descend en dessous du plateau distributeur **4** jusqu'à la pompe de recyclage **14**. Cette façon d'opérer assure une mise en mouvement contrôlée du liquide vers le haut et à travers le lit catalytique **12** ; toutefois une recirculation au moyen de conduits externes au réacteur est également possible, ainsi qu'une combinaison des deux procédés.

L'espace **8**, situé à l'intérieur du réacteur **5** au dessus du niveau de liquide **9**, est rempli par la phase gazeuse. Une fraction de tête contenant du liquide et du gaz est soutirée au moyen de la ligne **7** est envoyé dans le séparateur haute pression **18**. La fraction gazeuse récupérée contient essentiellement du gaz de synthèse et des hydrocarbures légers. Cette fraction est envoyée par la conduite **22** dans l'échangeur **23**, puis après refroidissement dans le séparateur gaz/liquide **24**. La fraction gazeuse obtenue en sortie du séparateur **24** est envoyée par la conduite **27** dans une unité de purification du gaz **28**, incluant généralement au moins une boîte froide, avec purge éventuelle **29**.

Après purification, le gaz **30** obtenu contient essentiellement du gaz de synthèse et peut être recomprimé au moyen du compresseur **31**, puis recyclé par la conduite **32** vers l'alimentation en gaz de synthèse du réacteur.

La phase liquide **19** récupérée en sortie du séparateur haute pression **18** est décomprimée en **20**, puis envoyée dans la colonne de distillation atmosphérique **33** par la conduite **21**. De même, la fraction liquide obtenue en **25**, après le séparateur gaz/liquide **24**, est décomprimée en **26**, puis envoyée dans la colonne de distillation atmosphérique **33**.

La fraction gazeuse **34**, issue de la distillation atmosphérique **33**, est envoyée dans un séparateur **40**. Cette séparation permet d'obtenir un gaz à basse pression **41**, ainsi qu'une phase liquide **35** servant de reflux dans la colonne de fractionnement **33**, et de produire une coupe naphta **42**.

La distillation atmosphérique permet également de produire une coupe kérosène en **36**, ainsi qu'une ou éventuellement plusieurs coupes gasoil en **37**, et des paraffines lourdes en **38**. Une partie de la coupe gasoil obtenue en **37** est recyclée au réacteur **5** via la ligne **17**, et une autre partie soutirée via la conduite **39**.

L'exemple qui suit illustre l'invention.

### EXEMPLE

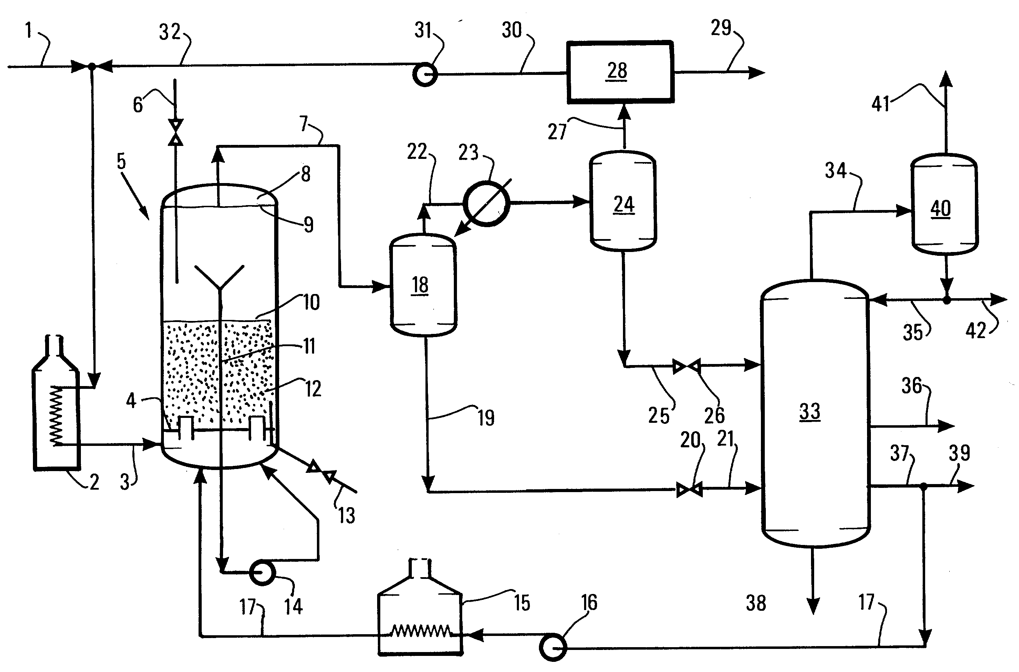

Un catalyseur à base de cobalt, molybdène et ruthénium déposés sur silice est chargé dans le réacteur représenté figure 1. Ce catalyseur comprend 20 % poids de cobalt, 0,8 % poids de ruhénium et 0,2 % poids de cuivre. ll a été préparé selon le protocole décrit dans le brevet US-A-5.302.622.

Une phase liquide issue de la synthèse Fischer-Tropsch, constituée par des hydrocarbures paraffiniques contenant 15 à 40 atomes de carbone (coupe gasoil légère), est introduite dans le réacteur et recyclée.

Ce catalyseur est réduit en phase liquide à pression atmosphèrique par un mélange hydrogène azote contenant 6 % volume d'hydrogène dans l'azote, puis par de l'hydrogène pur jusqu'à 350°C.

La phase liquide recyclée dans le réacteur est alors issue du fractionnement de l'effluent issu du réacteur de synthèse (cf. figure 1, coupe gasoil).

Le tableau 1 présente les performances obtenues dans ces conditions.

**Tableau 1 :**

| **CONVERSION DU GAZ DE SYNTHESE EN HYDROCARBURES** | |
|---|---|
| Température (°C) | 240 |
| Pression (MPa) | 2 |
| H₂/CO (Moles) | 2 |
| % CO₂ (Moles) | 3 |
| VVH* gaz (l/l cata h-¹) | 700 |
| VVH* liquide (l/l cata h-¹) | 0,32 |
| Conversion CO (% volume) | 80 |
| Distribution des hydrocarbures (% poids) | |
| Méthane | 7,6 |
| Hydrocarbures C₂-C₄ | 3,2 |
| Hydrocarbures C₅⁺ | 89,2 |

| | |
|---|---|
| * Vitesse volumique horaire. | |

Le procédé en phase liquide selon l'invention permet donc de limiter la formation de méthane et d'atteindre une conversion et une sélectivité en hydrocarbure C₅⁺ élevées.

## Revendications

1. Procédé de synthèse d'hydrocarbures essentiellement linéaires et saturés, contenant au moins 80 % poids d'hydrocarbures C₅⁺ par rapport à l'ensemble des hydrocarbures formés, à partir d'une charge comprenant du monoxyde de carbone CO, de l'hydrogène H₂ et éventuellement du bioxyde de carbone CO₂, ledit procédé étant caractérisé en ce que l'on opère dans une zone réactionnelle en lit catalytique bouillonnant en présence d'un catalyseur comprenant au moins un métal du groupe VIII et en présence d'une phase liquide.

2. Procédé selon la revendication 1 tel que le catalyseur est soumis à une préréduction avant utilisation, ladite préréduction du catalyseur étant effectuée par mise en contact avec un mélange de gaz inerte et d'au moins un composé réducteur en rapport molaire composé réducteur/(composé réducteur + gaz inerte) compris entre 0,001:1 et 1:1, la préréduction étant menée entre 150°C et 600°C, de préférence entre 200 et 500°C, entre 0,1 et 10 MPa et à une vitesse volumétrique horaire de 100 à 40 000 volumes de mélange par volume de catalyseur et par heure.

3. Procédé selon l'une des revendications 1 ou 2 tel que la conversion du gaz de synthèse en hydrocarbures est opérée sous une pression totale comprise entre 0,1 et 15 MPa, la température est comprise entre 150 et 350 °C, la vitesse volumique horaire est comprise entre 100 et 30 000 volumes de gaz de synthèse par volume de catalyseur et par heure, le rapport molaire H₂/CO dans le gaz de synthèse est compris entre 1:2 et 5:1 et la vitesse de liquide est comprise entre 0,1 et 10 volume de liquide par volume de catalyseur et par heure.

4. Procédé selon l'une des revendications 1 à 3 dans lequel ladite phase liquide est une phase liquide hydrocarbonée.

5. Procédé selon l'une des revendications 1 à 4 dans lequel la phase liquide comprend au moins un hydrocarbure ayant 5 atomes de carbone par molécule.

6. Procédé selon l'une des revendications 1 à 5 dans lequel la préréduction du catalyseur est effectué en présence d'une phase liquide comprenant au moins un hydrocarbure ayant au moins 5 atomes de carbone par molécule.

7. Procédé selon l'une des revendications 1 à 6 tel que l'on recycle au moins une partie de l'effluent, éventuellement après fractionnement, en zone réactionnelle.

8. Procédé selon la revendication 7 tel que le rapport entre le volume de liquide recyclé et le volume de charge est compris entre 0,5:1 et 50:1.

9. Procédé selon l'une des revendications 1 à 8 tel que le catalyseur est ajouté à la charge.

10. Procédé selon l'une des revendications 1 à 9 tel que le catalyseur est périodiquement soutiré de la zone réactionnelle tandis que du catalyseur frais est ajouté dans ladite zone.

11. Procédé selon l'une des revendications 1 à 10 tel que ledit métal du groupe VIII est choisi dans le groupe formé par le fer et le cobalt.

12. Procédé selon l'une des revendications 1 à 11 tel que ledit métal du groupe VIII est le cobalt.
